# EUROPÄISCHE PATENTSCHRIFT

(11) **EP 2 091 456 B1**
(45) Veröffentlichungstag und Bekanntmachung des Hinweises auf die Patenterteilung: **10.07.2013**
(21) Anmeldenummer: 07846584.6
(22) Anmeldetag: 14.11.2007
(51) Int. Cl.: A61B 18/20, A61N 5/06

(54) **VORRICHTUNG ZUR HAUTBEHANDLUNG**
DEVICE FOR TREATING THE SKIN
DISPOSITIF DE TRAITEMENT CUTANÉ

(30) Priorität: 18.11.2006 DE 102006054468
(43) Veröffentlichungstag der Anmeldung: 26.08.2009
(73) Patentinhaber: Braun GmbH, 61476 Kronberg/Taunus (DE)
(72) Erfinder: BOGATIRSKY, Dmitri, 60487 Frankfurt/Main (DE); BEERWERTH, Frank, 65558 Kaltenholzhausen (DE); HARTTMANN, Brigitte, 65527 Niedernhausen (DE)
(86) Internationale Anmeldenummer: PCT/EP2007/009831
(87) Internationale Veröffentlichungsnummer: WO 2008/058716

(56) Entgegenhaltungen:
- WO-A-2004/037287
- WO-A-2004/080279
- WO-A-2005/009266
- WO-A-2005/092438
- WO-A-2005/112815
- WO-A-2006/122136
- WO-A-2006/134426
- WO-A1-02/22031
- WO-A2-03/073058
- WO-A2-2006/089227
- DE-A1- 10 120 787
- US-A- 3 233 461
- US-A1- 2002 062 142

## Beschreibung

Die vorliegende Erfindung betrifft eine Vorrichtung zur Wuchsbeeinflussung und/oder Entfernung von Körperhaar, mit einem Arbeitskopf, der einen Strahlungsgeber zur Bestrahlung der zu behandelnden Hautoberfläche mit elektromagnetischer Strahlung, vorzugsweise mit Licht, aufweist, sowie eine den Strahlungsgeber umgebende Auflagefläche zum Auflegen des Arbeitskopfes auf die Hautoberfläche besitzt, sowie mit einer Sicherheitseinrichtung zur Steuerung der Bestrahlung und/oder Bereitstellung eines Sicherheitssignals, die eine Erfassungseinrichtung zur Erfassung der Auflage des Arbeitskopfes auf der Hautoberfläche aufweist.

Bei einer Vorrichtung der genannten Art wird elektromagnetische Strahlung auf die Haut appliziert, um etwa eine Hautverjüngung oder Glättung zu bewirken, oder die Strahlung wird insbesondere von Haarfollikeln absorbiert, was zu einer zeitweisen oder permanenten Reduktion des Haarwuchses aufgrund einer thermischern Schädigung der Haarfollikel führt.

Zur Entfernung von Körperhaar werden neben den üblichen mechanisch wirkenden Epilierern zunehmend Geräte zur Entfernung von Körperhaar eingesetzt, die die Hautoberfläche insbesondere mit elektromagnetischer Strahlung einer geeigneten Wellenlänge, insbesondere Laserlicht oder gepulstem Licht, bestrahlen, um die in der Haut eingebetteten bzw. unter der Haut liegenden Haarwurzeln und -follikel zu beschädigen. Hierbei kann insbesondere mit Wellenlängen im Bereich von 600 bis 1100 nm und Pulslängen im Bereich vom 20 bis 500 ms gearbeitet werden, so dass das Haar und die unmittelbar umliegenden Gewebeschichten einer Photothermolyse unterworfen werden, durch die die Struktur der Haar- und - follikelzellen verändert und das Haarwachstum reduziert werden kann.

Da hierbei mit hohen Energiedichten im Bereich von 1 bis 50 J/cm² gearbeitet wird, ist eine sachgemäße Anwendung erforderlich. Derartige Heimgeräte bedürfen daher besonderer Sicherheitsmaßnahmen.

Dies bezüglich wurden bereits verschiedene Sicherheitsmaßnahmen vorgeschlagen. So beschreiben die US 2005/0049657 und die US 2005/0045189 jeweils Gerätschaften zur Bestrahlung von Körperhaut bzw. zur Reduzierung von Körperhaar, bei denen die Bedienperson verschiedene Geräteparameter einstellen kann, um die Bestrahlung an die persönlichen Voraussetzungen anzupassen. Um gefährliche Fehlbedienungen zu verhindern, umfassen die Geräte eine Sicherheitseinrichtung, die bei entsprechender Einstellung eines Geräteparameters dazu führen, dass für andere Geräteparameter entsprechende Obergrenzen nicht überschritten werden können, um die insgesamt applizierte Energieinhalt (insbesondere pro Zeit und pro Fläche) zu beschränken. Aus der US 2005/0177139 A1 ist ein Gerät zur Entfernung von Körperhaar bekannt, bei dem mittels einer als Geschwindigkeitssensor dienenden Walze, die auf der Hautoberfläche abrollt, die Bewegungsgeschwindigkeit erfasst wird, mit der der Arbeitskopf über die Hautoberfläche bewegt wird. In Abhängigkeit von der erfassten Bewegungsgeschwindigkeit wird die Bestrahlungsintensität verändert, insbesondere wird bei zu langsamem Bewegen des Arbeitskopfes die Bestrahlungsintensität reduziert, um eine übermäßige Bestrahlung der Hautoberfläche zu vermeiden. Eine ähnliche Sicherheitseinrichtung beschreibt die US 2006/0020260, die ebenfalls mittels eines Geschwindigkeitssensors die Bewegungsgeschwindigkeit des Arbeitskopfes erfasst und die Laserstrahlungsquelle entsprechend steuert. Der entsprechende Sensor soll dabei vorzugsweise optisch arbeitend ausgebildet sein und gleichzeitig die Nähe der Haut zum Sensor erfassen, so dass die Laserquelle abgeschaltet werden kann, sobald der Arbeitskopf mit dem Sensor von der zu behandelnden Haut zu weit weg bewegt wird. Ein Haarentfernungs- bzw. -reduzierungsgerät mit einer ähnlichen Sicherheitseinrichtung ist auch aus der WO 2006/005443 bekannt.

Aus der WO 2005/009266 A1 ist eine Vorrichtung zur Applikation von Licht auf die Haut bekannt, die einen Behandlungskopf mit einer Vertiefung, aus der das Licht emittiert wird und in welcher der Luftdruck durch eine Pumpe verringert werden kann, aufweist. Die Vorrichtung weist auch eine Druckmesseinheit auf, die den Luftdruck in der Vertiefung misst. Oberhalb eines Luftdruckschwellwertes wird die Lichtemission von einem Kontrollmittel nicht aktiviert, da dies bedeutet, dass das Gerät nicht dicht auf der zu bestrahlenden Hautoberfläche aufliegt. Dass dabei die Haut in die Vertiefung hineingesaugt wird, führt auch zu einer Verbesserung der Eigenschaften der Haut bezüglich der Absorption von Lichtstrahlung.

Hiervon ausgehend liegt der vorliegenden Erfindung die Aufgabe zugrunde, eine verbesserte Vorrichtung zu schaffen, die Nachteile des Standes der Technik vermeidet und letzteren in vorteilhafter Weise weiterbildet. Vorzugsweise soll mit einer einfach aufgebauten Sicherheitseinrichtung eine erhöhte Bediensicherheit erreicht werden, die auch den Heimgebrauch der Vorrichtung gefährdungsfrei ermöglicht. Vorzugsweise soll die Sicherheitseinrichtung einen problemlosen Einsatz auch an unregelmäßig geformten, knochigen Körperoberflächen ermöglichen und die Bearbeitungsfläche des Arbeitskopfes nicht zu stark beschränken, um einen effizienten Gebrauch durch große Bearbeitungsflächen ermöglichen.

Erfindungsgemäß wird diese Aufgabe durch eine Vorrichtung gemäß Anspruch 1 gelöst. Weitere Ausgestaltungen der Erfindung sind Gegenstand der abhängigen Ansprüche.

Es wird also vorgeschlagen, zur Überwachung des korrekten Aufsetzens des Arbeitskopfes auf die zu behandelnde Oberfläche die Schalldurchlässigkeit der den Strahlungsgeber umgebenden Auflagefläche zu überprüfen. Hierbei wird von dem Gedanken ausgegangen, dass bei einem Spalt zwischen der Auflagefläche des Arbeitskopfes und der Hautoberfläche Schall leicht aus- bzw. eintreten kann. Hingegen kann der Schall bei einem dichten Aufliegen nicht ohne weiteres aus- bzw. eintreten. Dabei ist eine Spaltbildung zwischen der Auflagefläche und der zu behandelnden Haut gleichzeitig das Kriterium für eine Früherkennung einer möglichen Gefährdung, da bei einem Spalt elektromagnetische Strahlung, insbesondere intensives Laserlicht austreten kann, was hingegen bei einem dichten Aufliegen nicht der Fall ist. Erfindungsgemäß ist vorgesehen, dass die Auflagefläche des Arbeitskopfes einen insbesondere glockenartig auf die Hautoberfläche setzbaren Schallraum begrenzt und die Erfassungseinrichtung eine Dichtheits-Sensoreinrichtung zur Bestimmung der Schalldichtheit des Schallraums aufweist. Der Arbeitskopf ist also um den Strahlungsgeber herum sozusagen als Schallglocke ausgebildet, die zusammen mit der darauf aufliegenden Hautoberfläche einen Raum begrenzt, der gegenüber der Umgebung schallreduzierend wirkt, wobei es bereits ausreichend sein kann, wenn die isolierende Wirkung nur in begrenztem Maße vorhanden ist. Durch eine Messung des Grades der Schalldichtheit des von der Auflagefläche umschlossenen Raumes kann bestimmt werden, ob der Arbeitskopf korrekt auf der Haut aufsitzt und dementsprechend kann die Bestrahlung der Haut mit elektromagnetischer Strahlung in Abhängigkeit vom dichten Aufliegen der Auflagefläche gesteuert werden. Die verwendete elektromagnetische Strahlung kann insbesondere Strahlung im sichtbaren Lichtspektrum, im IR-Bereich oder im UV-Bereich sein. Ein glockenartiger Schallraum soll nicht bedeuten, dass der Raum eine glockenförmige Form hat. Auch ein Raum, der durch einen ebenen Arbeitskopf, durch eine den ebenen Arbeitskopf umgebende, vorstehende Auflagefläche (die etwa in Form eines geschlossenen mehreckigen oder ovalen bzw. runden Rings ausgeformt ist) und die Haut gebildeter Raum soll von "glockenartig" bzw. vom den Begriff "Schallglocke" umfasst sein.

Die Dichtheits-Sensoreinrichtung weist eine akustische Sensorik auf, mittels derer der Schalldurchgang durch einen Spalt zwischen Hautoberfläche und Auflagefläche bzw. einem dort eben nicht vorhandenen Spalt erfasst werden kann. Hierzu besitzt die Dichtheits-Sensoreinrichtung vorteilhafterweise auf einer Seite der Auflagefläche zumindest einen Schallerzeuger und auf einer gegenüberliegenden Seite der Auflagefläche zumindest einen Schalldetektor. Hierdurch kann präzise bestimmt werden, in welchem Maße die den Strahlungsgeber umgebende Schallglocke schalldicht ist. Tritt ein Spalt auf, kann Schall leicht aus- bzw. eintreten, während bei korrekt aufliegender Auflagefläche der Schalldurchtritt entsprechend behindert ist.

Grundsätzlich kann die Anordnung des zumindest einen Schallerzeugers und des zumindest einen Schalldetektors unterschiedlich getroffen sein. Beispielsweise könnte der Schallerzeuger innerhalb des von der Auflagefläche umschlossenen Raumes angeordnet sein, so dass mit einem außerhalb angeordneten Schalldetektor der austretende Schall erfasst werden kann. In bevorzugter Weiterbildung der Erfindung jedoch ist der zumindest eine Schallerzeuger außerhalb des Schallraums angeordnet, während innerhalb desselben der zumindest eine Schalldetektor angeordnet ist. Hierdurch können Umgebungsgeräusche und Hintergrundschall in ihrem Einfluss deutlich reduziert werden. Durch die Anordnung des Schalldetektors innerhalb des Schallraumes kann exakt erfasst werden, in welchem Maße Schall durch einen ggf. bestehenden Spalt eindringen kann. Sitzt der Arbeitskopf korrekt auf der zu behandelnden Hautoberfläche, tritt ein deutlich spürbarer Abfall der Schallamplitude des von dem Schallerzeuger erzeugten Schalls auf, während der Amplitudenabfall deutlich reduziert sein wird, wenn zwischen der Auflagefläche des Arbeitskopfes und der zu behandelnden Hautoberfläche ein Spalt ist. Gegebenenfalls könnte auf einen Schallerzeuger auch gänzlich verzichtet werden, da bei ausreichenden Umgebungs- bzw. Hintergrundgeräuschen der in den Druck- bzw. Schallraum eintretende Umgebungslärm erfasst werden kann. Bevorzugt ist jedoch die zuvor beschriebene Ausbildung mit Anordnung eines Schallerzeugers außerhalb der Auflagefläche, da hiermit unabhängig von Hintergrundgeräuschen mit hoher Präzision das Auftreten eines Spalts erfasst werden kann.

Grundsätzlich ist hierbei ein Schallerzeuger ausreichend. In einer Ausführung der Erfindung jedoch können mehrere Schallerzeuger an verschiedenen Abschnitten der Auflagefläche angeordnet sein. Über mehrere über den Umfang der Auflagefläche verteilte Schallgeber können an verschiedenen Stellen auftretende Spalte mit hoher Unterscheidungskraft einfach und präzise bestimmt werden.

In einer Weiterbildung sind die Schallgeber auf einen Rand der Auflagefläche gerichtet und geben den Schall gezielt auf den Rand der Auflagefläche ab.

Die Auswertung der Signale der Schallerfassungseinrichtung kann grundsätzlich verschieden erfolgen. Beispielsweise könnte ein Absolutwert des über die Auflagefläche hinweg tretenden Schallpegels erfasst werden, wobei dieser Absolutwert dann mit einem entsprechenden Sollwert verglichen wird, um zu entscheiden, ob die Auflagefläche korrekt und dicht auf der Hautoberfläche aufsitzt oder nicht. In einer Weiterbildung der Erfindung ist die Auswerteeinheit dazu vorgesehen, eine Änderung des Schallsignals zu überwachen und anhand der Quantität und/oder Qualität einer auftretenden Änderung zu entscheiden, ob ein die Sicherheit der Geräteanwendung gefährdendes Abheben des Arbeitskopfes auftritt. Insbesondere kann hierzu die Auswerteeinheit einen auftretenden Schallpegelanstieg bzw. -abfall betragsmäßig mit entsprechenden Schwellwerten vergleichen. Tritt eine übermäßige Schallpegeländerung auf, gibt die Auswerteeinheit ein Sicherheitssignal ab, welches angibt, dass ein die Bestrahlungssicherheit gefährdendes Abheben des Arbeitskopfes vorliegt.

Das aus der Schalldichtheit prüfung abgeleitete Sicherheitssignal kann in verschiedener Weise Verwendung finden. In einfacher Ausbildung der Erfindung kann ein beispielsweise akustisches, visuelles und/oder mechanisches, vorzugsweise Vibrationssignal abgegeben werden, welches dem Bediener anzeigt, dass der Arbeitskopf korrekt (oder nicht korrekt) über die Hautoberfläche geführt wird. In einer Weiterbildung der Erfindung ist die Sicherheitseinrichtung mit einer Steuereinrichtung verbunden, die die von dem Strahlungsgeber abgegebene Strahlung automatisch in Abhängigkeit eines Signals der Sicherheitseinrichtung steuert, insbesondere reduziert oder gänzlich abschaltet, wenn ein nicht bestimmungsgemäßer Einsatz des Arbeitskopfes detektiert wird. So wird etwa die Bestrahlung reduziert bzw. abgeschaltet, sobald die vorgenannte Dichtheits-Sensoreinrichtung eine entsprechende Schallpegeländerung erfasst. Insbesondere kann die Steuereinrichtung in Abhängigkeit eines entsprechenden Signals der Dichtheitssensoreinrichtung die Strahlungsquelle sofort abschalten.

Um ein fehlerfreies Funktionieren der Sicherheitseinrichtung auch bei der Bearbeitung schwieriger Körperoberflächen mit größeren Konturunebenheiten zu ermöglichen, ist nach einer Ausführung der Erfindung die Auflagefläche des Arbeitskopfes nachgiebig, insbesondere druckelastisch nach Art eines Kissens oder Balgs ausgebildet. Insbesondere kann die Auflagefläche zumindest teilweise von einem vorstehenden Ring gebildet sein, der eine konturanpassbare Stirnseite besitzt. Hierdurch kann die Auflagefläche auch bei uneben geformten Körperoberflächen spaltfrei auf die Hautoberfläche gesetzt und über diese hinweggeführt werden. Solche für Geräte zur Entfernung von Körperhaar schwierige Körpergliedoberflächen sind beispielsweise knöchrige Partien wie die Handrücken-/Handgelenksoberfläche oder die Schienbeinpartie. Die kissenartige, druckelastische Ausbildung der Auflagefläche das Arbeitskopfes ermöglicht hierbei nicht nur, das unerwünschte und ggf. gefährliche Austreten von Strahlung an solchen Körperpartien zu verhindern, sondern bewirkt auch ein zuverlässiges Funktionieren der zuvor beschriebenen Sicherheitseinrichtung bei der Bearbeitung solcher schwieriger Körperpartien, da die formveränderliche Ausbildung der Auflagefläche auch dort bei korrektem Ansetzen des Arbeitskopfes den in dem Schallraum herrschenden Druck halten bzw. den Schallübergang reduzieren kann. Ein weiterer Vorteil liegt darin, dass die Auflagefläche einen relativ großen Raum umschließen kann, ohne dass bei Körperflächenunebenheiten gleich jedes Mal eine Spaltbildung auftreten würde, da die druckelastische Ausbildung der Auflagefläche auch größere Unebenheiten ausgleiche kann. Dies ermöglicht es, die Bestrahlungsfläche relativ groß auszubilden, wodurch eine effiziente Bearbeitung bzw. Behandlung ermöglicht wird. Das Kissen kann dabei als eckig ausgeformter Ring (etwa dreieckig, rechteckig, fünfeckig etc.) oder als runder, insbesondere kreisrunder oder ovaler Ring ausgebildet sein. Das Kissen kann beispielsweise aus einem flexiblen Schaum, etwa einem Polyurethanschaum, hergestellt sein und kann auch eine Hülle aus einem elastischen, wasserdichten Material aufweisen. Das Kissen kann auch hohl ausgebildet sein, etwa indem die Auflagefläche aus einem Elastomer-Material, etwa Latex, hergestellt ist. Der Innenraum des Kissens kann über Öffnungen mit dem Schallraum verbunden sein und so Teil des Schallraums sein.

Um eine weitere Erhöhung der Sicherheit des Geräteinsatzes zu erreichen, kann die Sicherheitseinrichtung weiterhin einen Hauterfassungssensor aufweisen, mittels dessen erfasst werden kann, ob die auf der Auflagefläche des Arbeitskopfes aufliegende Oberfläche eine Hautoberfläche ist. Erfasst der Hauterfassungssensor beispielsweise, dass der Arbeitskopf nicht auf einer Hautoberfläche aufgesetzt ist, kann ein Sicherheitssignal bereitgestellt werden und/oder die vorgenannte Steuereinrichtung die Bestrahlung reduzieren bzw. abschalten, um eine nicht bestimmungsgemäße Oberflächenbestrahlung zu verhindern. Beispielsweise kann hierdurch unterbunden werden, dass Textil- oder Kleidungsoberflächen beschädigt werden, wenn mit dem Arbeitskopf über eine solche Fläche gefahren wird. Der Hauterfassungssensor kann vorteilhafterweise berührungslos arbeitend ausgebildet sein, insbesondere kann ein kapazitiver und/oder optischer Sensor als Hauterfassungssensor vorgesehen sein.

Alternativ oder Zusätzlich kann in Weiterbildung der Erfindung ein Bewegungssensor, insbesondere ein Geschwindigkeitssensor, vorgesehen sein, mit Hilfe dessen die Bewegung, insbesondere die Geschwindigkeit, erfasst wird, mit der der Arbeitskopf über die zu bearbeitende Oberfläche hinweggeführt wird. Dieser Bewegungssensor kann grundsätzlich verschieden ausgebildet sein, beispielsweise kann er mechanisch arbeiten und ein Tastrad umfassen. In alternativer Weiterbildung der Erfindung kann der Bewegungssensor auch optisch und/oder kapazitiv arbeitend ausgebildet sein. Der Bewegungssensor erfasst dabei in Weiterbildung der Erfindung nach Art einer PC-Maus die Bewegung des Arbeitskopfes über die auf dessen Auflagefläche sitzende Oberfläche. Der Bewegungssensor kann auch mittels Radarwellen oder Ultraschall arbeiten und insbesondere Messungen nach dem Doppler-Prinzip durchführen.

Das Bewegungs-, insbesondere Geschwindigkeitssignal kann von der Steuereinrichtung vorteilhafterweise dazu verwendet werden, um automatisch die von dem Strahlungsgeber abgestrahlte Bestrahlung zu steuern, beispielsweise kann mit abnehmender Geschwindigkeit und/oder bei Unterschreiten einer Mindestgeschwindigkeit die Energiedichte der Bestrahlung herabgefahren werden, um Verletzungen zu verhindern. Alternativ oder zusätzlich kann die Bestrahlungsintensität mit zunehmender Geschwindigkeit erhöht werden. Insbesondere kann die Bestrahlung gänzlich unterbunden werden, wenn überhaupt keine Bewegung mehr erfasst wird.

Der Arbeitskopf der Vorrichtung kann in Weiterbildung der Erfindung ein separates Bauteil bilden, das mit einem Basisteil der Vorrichtung über eine Energieleitung insbesondere in Form eines Lichtleitkabels verbunden ist. Hierdurch kann der Arbeitskopf kleinbauend ausgeführt werden, wodurch sich die Handhabung vereinfacht. Die Bestrahlungsquelle insbesondere in Form einer Laserquelle kann in dem Basisteil angeordnet sein, von dem aus das erzeugte Licht über das Lichtleitkabel in den Arbeitskopf geleitet wird, von dem aus das Licht über den vorgenannten Bestrahlungsgeber abgegeben wird. In alternativer Weiterbildung der Erfindung kann der Arbeitskopf jedoch auch einen Abschnitt des Gesamtgeräts bilden, d.h. die Bestrahlungsquelle insbesondere in Form einer Lasereinheit kann auch in den Arbeitskopf integriert sein.

In einer Weiterbildung der Erfindung weist die Vorrichtung einen Flüssigkeitsapplikator zur Applikation von Flüssigkeit auf die Haut auf. Ein solcher Flüssigkeitsapplikator wird etwa in der WO 2005/110266 A2 beschrieben.

Die Erfindung betrifft auch einen auswechselbaren Aufsatz für die beschriebene Vorrichtung, wobei der Aufsatz zumindest die Auflagefläche, die den Druck- und/oder Schallraum begrenzt, umfasst. Dieser Aufsatz kann auf den Arbeitskopf aufgesteckt werden, um die beschriebene Vorrichtung zu bilden. Ein solcher Aufsatz bietet den Vorteil, dass etwa für jeden Benutzer ein eigener Aufsatz vorgesehen sein kann, was aus hygienischen Gründen sinnvoll ist, oder dass unterschiedliche Aufsätze mit unterschiedlichen Geometrien der Auflagefläche vorgesehen sind, um sich den Gegebenheiten der zu behandelnden Hautoberfläche noch besser anzupassen.
- Fig. 1:: eine schematische, perspektivische Darstellung des Arbeitskopfes und des damit verbundenen Basisteils einer Vorrichtung zur Reduzierung von Körperhaar, bei der die Sicherheitseinrichtung eine Druckerfassungseinrichtung am Arbeitskopf aufweist, und
- Fig. 2:: eine schematische, perspektivische Darstellung des Arbeitskopfes und des damit verbundenen Basisteils einer Vorrichtung zur Reduzierung von Körperhaar nach einer bevorzugten Ausführung der Erfindung, bei der eine Schallerfassungseinrichtung am Arbeitskopf vorgesehen ist.

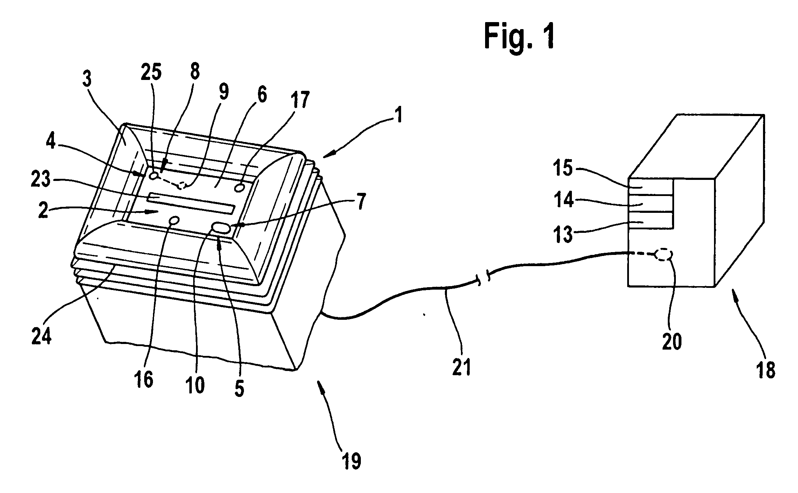

Das in Figur 1 gezeigte Bestrahlungs-Epiliergerät umfasst ein Basisteil 18 sowie ein Handteil 19, das einen Arbeitskopf 1 umfasst bzw. als Arbeitskopf 1 ausgebildet ist, vgl. Figur 1.

Dabei ist in dem Basisteil 18 eine Lichtquelle 20 insbesondere in Form einer Laserquelle angeordnet, deren Lichtstrahlung über einen Lichtleiter 21 zu dem Handteil 19 geleitet wird, das mit dem Basisteil 18 durch den genannten Lichtleiter 21 verbunden ist. Alternativ zu dieser Konfiguration könnte die Lichtquelle 20 auch in den Handteil 19 integriert sein bzw. der Handteil 19 mit dem Basisteil 18 zu einem gemeinsamen Gerätebaustein zusammengefasst sein.

An dem Arbeitskopf 1 wird das eingespeiste bzw. erzeugte Licht über einen Strahlungsgeber 2 abgegeben, der entsprechende optische Elemente umfassen kann und in der gezeichneten Ausführung einen bevorzugt schlitzförmigen Lichtaustrittsbereich 23 umfasst, der auch in anderen Austrittsgeometrien ausgebildet sein kann, über den die Laserstrahlung abgegeben und auf die zu bearbeitende Haut gestrahlt werden kann.

Der genannte Lichtaustrittsbereich 23 wird von einem wulstförmigen geschlossenen Ring 24 umgeben, der insgesamt betrachtet eine vorstehende Kontur definiert und in seinem Innenraum, in dem der Lichtaustrittsbereich 23 angeordnet ist, den Druck- und/oder Schallraum 6 begrenzt, der glockenartig auf die zu bearbeitende Hautoberfläche setzbar ist. Die Stirnseite des Rings 24 bildet dabei eine Auflagefläche 3, mit der der Arbeitskopf 1 bestimmungsgemäß auf die zu bearbeitende Hautoberfläche aufzusetzen ist. Die besagte Auflagefläche 3 ist dabei druckelastisch und konturanpassbar ausgebildet, so dass sie sich an reliefartig konturierte Körperpartien anpassen kann. In der unverformten Ausgangsstellung der gezeigten Ausführungsform definiert die Auflagefläche 3 eine ebene Fläche, die sich jedoch an Berge und Täler in der Körperoberfläche anpassen kann. Alternativ kann die Auflagefläche 3 aber auch eine konkave oder konvexe Fläche definieren. Da die viele geometrisch problematische Körperpartien konvex oder konkav geformt sind (insbesondere die Beine, die Knöchelregionen oder der Unterarmbereich), kann eine konkave bzw. konvexe Auflagefläche 3 ein dichtes Aufliegen auf solchen konvexen bzw. konkaven Hautflächen unterstützen. Der Ring 24 kann dabei als abnehmbarer Aufsatz ausgestaltet sein, sodass ein Benutzer je nach Anwendung etwa zwischen einem Ring 24 mit einer ebenen Auflagefläche 3 und einem Ring 24 mit einer konkaven oder konvexen Auflagefläche 3 wählen kann. Der Ring 24 kann hierzu insgesamt nach Art einer elastischen Wurst ausgebildet sein. In dem in Figur 1 gezeichneten Beispiel ist der Basisabschnitt des Rings 24 nach Art eines Faltenbalgs ausgebildet, vgl. Figur 1. Alternativ oder zusätzlich kann der stirnseitige Abschnitt des Rings 24 aus einem druckelastischen, schaumstoffartig verformbaren Material ausgebildet sein. Der Ring 24 kann insbesondere einen inneren Hohlraum haben. Dieser innere Hohlraum kann durch innen am Ringwulst liegende Öffnungen mit dem Druck- und/oder Schallraum verbunden sein, sodass dieser innere Hohlraum des Rings 24 ein mit dem Druck- und/oder Schallraum verbundener Raum ist und daher ein Teil des Druck- und/oder Schallraumes ist. Der Ring kann aus einem Elastomer-Material hergestellt sein, um die notwendige Druckelastizität zu gewährleisten.

Um das Aufliegen der Auflagefläche 3 auf der zu behandelnden Körperpartie zu erfassen und eine Spaltbildung zwischen der Auflagefläche 3 und der Hautoberfläche zu erkennen, ist dem Arbeitskopf 1 eine Erfassungseinrichtung 5 zugeordnet, die eine Dichtheitssensoreinrichtung 7 umfasst. In der in Figur 1 gezeichneten Ausführung umfasst die besagte Dichtheitssensoreinrichtung 7 eine Druckbeaufschlagungseinrichtung 8 in Form eines Luftstromerzeugers 9, dessen Luftaustrittsöffnung 25 in Figur 1 zu sehen ist und innerhalb des Rings 24 in den Druck- bzw. Schallraum 6 mündet. Weiterhin ist in dem von dem Ring 24 umgrenzten Druck- bzw. Schallraum 6 ein Drucksensor 10 angeordnet, mittels dessen der sich durch den zugeführten Luftstrom aufbauende Überdruck erfasst werden kann. Alternativ zu der gezeigten Ausführungsform kann die Luftaustrittsöffnung 25 und/oder der Drucksensor 10 unterhalb eines hohl ausgeformten Rings 24 angeordnet sein. Dabei kann Luft dann aus innen am Ring 24 liegenden Öffnungen (nicht gezeigt), die bei einem dichten Aufliegen typischerweise nicht durch die Haut bedeckt werden, in den Druck- und/oder Schallraum strömen, sodass der hohle Innenraum des Rings 24 Teil des Druck- und/oder Schallraums ist, da sich bei einem dichten Aufliegen ein Druckgleichgewicht zwischen dem hohlen Innenraum und dem vom Ring 24 umgebenen Raum einstellt.

Die Signale des Drucksensors 10 werden einer Auswerteeinheit 13 zugeführt, die die sich einstellenden Druckänderungen mit Hilfe einer Vergleichseinheit 14 mit zugehörigen Schwellwerten vergleicht. Wird ein zu großer Druckabfall festgestellt, der sich immer dann einstellt, wenn die Auflagefläche 3 nicht korrekt auf der Hautoberfläche aufliegt, wird von einer mit der Auswerteeinheit 13 verbundenen Steuereinrichtung 15 die Lichtquelle 20 unverzüglich herabgefahren (also in ihrer Intensität reduziert oder ausgeschaltet), um ein Gefährdungspotential durch die von dem Strahlungsgeber 2 abgegebene Strahlung auszuschließen. Der Drucksensor 10 kann dabei den im Druck- und/oder Schallraum 6 herrschenden Druck als Relativdruck zum Umgebungsdruck messen, sodass die Druckmessung nicht Änderungen eines Absolutdruckes detektieren muss und von den Umgebungsdruckverhältnissen unabhängig ist.

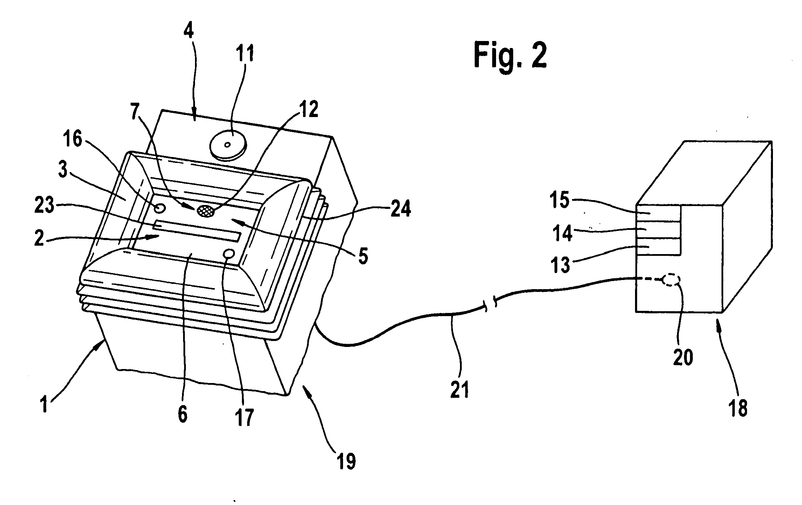

Erfindungsgemäß ist alternativ oder zusätzlich zu einer solchen Druck- bzw. Druckänderungserfassungseinrichtung dem Arbeitskopf 1 eine Schalldurchlässigkeits-Erfassungseinrichtung zugeordnet, wie sie die in Figur 2 dargestellte Ausführung des Arbeitskopfes 1 zeigt. Hierbei kann insbesondere außerhalb des Druck- und/oder Schallraumes 6, d.h. auf der Außenseite des Ringes 24, ein Schallerzeuger 11 am Arbeitskopf 1 angeordnet sein, während andererseits in dem Druck- und/oder Schallraum 6 ein Schalldetektor 12, beispielsweise in Form eines Mikrofons, vorgesehen ist. Vorteilhafterweise ist dabei der Schallerzeuger 11 so angeordnet, dass der von ihm erzeugte Schall auf die Auflagefläche 3 abgegeben wird. Obwohl Figur 2 nur einen solchen Schallerzeuger 11 zeigt, können in Weiterbildung der Erfindung mehrere Schallerzeuger 11 und/oder mehrere Schalldetektoren 12 vorgesehen, insbesondere über den Umfang des Ringes 24 verteilt angeordnet sein. Bei der in Figur 2 gezeichneten rechteckigen Ausbildung des Ringes 24 würde es sich anbieten, an jedem der vier Schenkel des Rings 24 einen Schallerzeuger 11 zu positionieren. Optional kann der zumindest eine Schallerzeuger 11 jedoch beispielsweise auch an dem Basisteil 18 angeordnet sein.

Der Schalldetektor 12 ist ebenfalls mit der Auswerteeinheit 13 der Steuereinrichtung 15 verbunden, die etwa mit Hilfe der Vergleichseinheit 14 den Schallpegelabfall zwischen dem Schallerzeuger 11 und dem Schalldetektor 12 mit entsprechenden Schwellwerten vergleicht. Sitzt die Auflagefläche 3 korrekt auf der zu behandelnden Haut, wird ein substantieller Amplitudenabfall von dem Schallerzeuger 11 zu dem Schalldetektor 12 hin auftreten, während dieser Amplitudenabfall deutlich geringer sein wird, wenn an der entsprechenden Auflagefläche 3 ein Spalt ist. Die Steuereinrichtung 15 kann auch in diesem Fall, wenn kein ausreichender Schallpegelabfall auftritt, die Lichtquelle 20 unmittelbar herunterfahren, um Gefährdungen zu vermeiden.

Weiterhin kann an dem Arbeitskopf 1 ein Hauterfassungssensor 16 vorgesehen sein, der etwa zwischen dem Lichtaustrittsbereich 22 und dem Ring 24 angeordnet wird, wie dies die Figuren 1 und 2 zeigen. Mit Hilfe des Hauterfassungssensors 16 wird festgestellt, ob die auf der Auflagefläche 3 aufliegende Oberfläche eine Hautoberfläche ist. Ist dies nicht der Fall, beispielsweise wenn mit dem Arbeitskopf 1 über ein Textil- bzw. Bekleidungsstück gefahren wird, kann die mit dem Hauterfassungssensor 16 verbundene Steuereinrichtung 15 ebenfalls die Lichtquelle 20 herabfahren (also in ihrer Intensität reduzieren oder ausschalten). Der besagte Hauterfassungssensor 16 kann kapazitiv arbeiten und/oder optisch ausgebildet sein.

Weiterhin zeigen die Figuren 1 und 2 einen Bewegungssensor 17, der nach Art einem PC-Maus-Sensor die Bewegung des Arbeitskopfes 1 über die zu behandelnde Oberfläche und insbesondere dessen Geschwindigkeit erfasst. Die mit dem Bewegungssensor 17 verbundene Steuereinrichtung 15 kann die Lichtquelle 20 in Abhängigkeit der erfassten Bewegungsgeschwindigkeit steuern, insbesondere die Lichtquelle 20 herabfahren, sobald eine Mindestgeschwindigkeit unterschritten wird. Der Bewegungssensor 17 kann ebenfalls verschieden ausgebildet sein, beispielsweise kann ein mechanisch arbeitender Sensor vorgesehen sein. Alternativ oder zusätzlich kann ein optisch arbeitender und/oder kapazitiv arbeitender Bewegungssensor 17 vorgesehen sein.

## Patentansprüche

1. Vorrichtung zur Wachstumsreduzierung und/oder Entfernung von Körperhaar mit einem Arbeitskopf (1), der einen Strahlungsgeber (2) zur Bestrahlung der zu behandelnden Hautoberfläche mit elektromagnetischer Strahlung, sowie eine den Bestrahlungsgeber (2) umgebende Auflagefläche (3) zum Auflegen des Arbeitskopfs (1) auf die Hautoberfläche aufweist, sowie mit einer Sicherheitseinrichtung (4) zur Steuerung der Bestrahlung und/oder Bereitstellung eines Sicherheitssignals, die eine Erfassungseinrichtung (5) zur Erfassung des Aufliegens des Arbeitskopfes (1) auf der Hautoberfläche aufweist, **dadurch gekennzeichnet, dass** die Auflagefläche (3) einen Schallraum (6) begrenzt und die Erfassungseinrichtung (5) eine Dichtheits-Sensoreinrichtung (7) zur Bestimmung der Schalldichtheit des Schallraums (6) aufweist.

2. Vorrichtung nach Anspruch 1, wobei die Dichtheits-Sensoreinrichtung (7) auf einer Seite der Auflagefläche (3) einen Schallerzeuger (11) und auf einer gegenüberliegenden Seite der Auflagefläche (3) einen Schalldetektor (12) aufweist.

3. Vorrichtung nach dem vorhergehenden Anspruch, wobei der Schallerzeuger (11) außerhalb des Schallraums (6) und der Schalldetektor (12) innerhalb des Schallraums (6) angeordnet ist.

4. Vorrichtung nach einem der beiden vorhergehenden Ansprüche, wobei der Schallerzeuger (11) auf einen Rand der Auflagefläche (3) gerichtet ist.

5. Vorrichtung nach einem der Ansprüche 2 bis 4, wobei mehrere Schallerzeuger (11) an verschiedenen Abschnitten der Auflagefläche (3) vorgesehen sind.

6. Vorrichtung nach einem der vorhergehenden Ansprüche, wobei die Dichtheits-Sensoreinrichtung (7) mit einer Auswerteeinheit (13) zur Bestimmung von Änderungen in der erfassten Dichtheit des Schallraums (6) verbindbar ist.

7. Vorrichtung nach dem vorhergehenden Anspruch, umfassend die Auswerteeinheit (13), wobei die Auswerteeinheit (13) zur Bestimmung eines Schallpegelanstiegs vorgesehen ist.

8. Vorrichtung nach dem vorhergehenden Anspruch, wobei die Auswerteeinheit (13) eine Vergleichseinheit (14) zum Vergleichen einer erfassten Änderung des Schallpegels mit einem vorbestimmten Schwellwert aufweist und bei Überschreiten dieses Schwellwerts ein Sicherheitssignal bereitstellt.

9. Vorrichtung nach einem der vorhergehenden Ansprüche, wobei die Sicherheitseinrichtung (4) mit einer Steuereinrichtung (15) zur Reduzierung und/oder Abschaltung der von dem Strahlungsgeber (2) abgegebenen Strahlung in Abhängigkeit eines Signals der Dichtheits-Sensoreinrichtung (7) verbindbar ist.

10. Vorrichtung nach einem der vorhergehenden Ansprüche, wobei die Auflagefläche (3)
nachgiebig, insbesondere druckelastisch, vorzugweise nach Art eines Kissens oder Balgs, ausgebildet ist.

11. Vorrichtung nach einem der vorhergehenden Ansprüche, wobei die Auflagefläche (3) zumindest teilweise von einem vorstehenden Ring mit einer konturanpassbaren Stirnseite gebildet ist.

12. Vorrichtung nach einem der beiden vorhergehenden Ansprüche, wobei die Dichtheits-Sensoreinrichtung (7) unterhalb der Auflagefläche (3) angeordnet ist.

13. Vorrichtung nach einem der vorhergehenden Ansprüche, wobei die Auflagefläche (3) Teil eines von dem Arbeitskopf (1) lösbaren Aufsatzes ist.

14. Vorrichtung nach einem der vorhergehenden Ansprüche, wobei die Erfassungseinrichtung (5) einen Hauterfassungssensor (16) zur Bestimmung der auf der Auflagefläche (3) aufliegenden Oberfläche dahingehend, ob es sich dabei um eine Hautoberfläche oder nicht um eine Hautoberfläche handelt, aufweist.

15. Vorrichtung nach dem vorhergehenden Anspruch, wobei der Hauterfassungssensor berührungslos arbeitend, insbesondere als ein kapazidv und/oder optisch arbeitender Sensor ausgebildet ist.

16. Vorrichtung nach einem der vorhergehenden Ansprüche, wobei ein Bewegungssensor (17), insbesondere ein Geschwindigkeitssensor, zur Erfassung der Bewegung, insbesondere der Geschwindigkeit, des Arbeitskopfes (1) über die zu behandelnde Oberfläche vorgesehen ist.

17. Vorrichtung nach dem vorhergehenden Anspruch, wobei der Bewegungssensor (17) mechanisch, optisch und/oder kapazitiv arbeitend ausgebildet ist.

18. Vorrichtung nach einem der vorhergehenden Ansprüche, wobei eine Steuereinrichtung zur Steuerung der von dem Strahlungsgeber (2) abgegebenen Bestrahlung in Abhängigkeit einer Bewegungsgröße des Arbeitskopfs (1) vorgesehen ist.

19. Vorrichtung nach einem der vorhergehenden Ansprüche, wobei die Vorrichtung einen Flüssigkeitsapplikator zur Applikation einer Flüssigkeit auf die Haut aufweist.

## Claims

1. A device for reducing the growth of and/or removing body hair with an operating head (1) which has a radiation transmitter (2) for radiating with electromagnetic radiation the surface of the skin to be treated, as well as a contact surface (3) surrounding the radiation transmitter (2) for placing the operating head (1) on the surface of the skin, as well as with a safety device (4) to control the radiation and/or provide a safety signal, which safety device (4) has a detection device (5) for detecting the placement of the operating head (1) on the surface of the skin, **characterized in that** the contact surface (3) confines a sound chamber (6), and the detection device (5) has an impermeability sensor device (7) for determining the sound impermeability of the sound chamber (6).

2. The device according to claim 1, wherein the impermeability sensor device (7) has a sound source (11) on one side of the contact surface (3) and a sound detector (12) on the opposite side of the contact surface (3).

3. The device according to the preceding claim, wherein the sound source (11) is positioned outside the sound chamber (6), and the sound detector (12) is positioned inside the sound chamber (6).

4. The device according to any one of the two preceding claims, wherein the sound source (11) is directed towards an edge of the contact surface (3).

5. The device according to any one of claims 2 to 4, wherein multiple sound sources (11) are provided on different sections of the contact surface (3).

6. The device according to any one of the preceding claims, wherein the impermeability sensor device (7) can be connected to an evaluation unit (13) for determining changes in the detected impermeability of the sound chamber (6).

7. The device according to the preceding claim, comprising the evaluation unit (13), wherein the evaluation unit (13) is provided to determine an increase in the sound level.

8. The device according to the preceding claim, wherein the evaluation unit (13) has a comparison unit (14) for comparing a detected change in the sound level with a predetermined threshold value, and emits a safety signal if this threshold value is exceeded.

9. The device according to any one of the preceding claims, wherein the safety device (4) can be connected to a control unit (15) to reduce and/or turn off the radiation emitted by the radiation transmitter (2) depending upon a signal from the impermeability sensor device (7).

10. The device according to any one of the preceding claims, wherein the contact surface (3) is designed to be flexible, in particular pressure-elastic, preferably like a cushion or bellows.

11. The device according to any one of the preceding claims, wherein the contact surface (3) is at least partially formed by a protruding ring with a front end which adapts to contours.

12. The device according to one of the two preceding claims, wherein the impermeability sensor device (7) is positioned below the contact surface (3).

13. The device according to any one of the preceding claims, wherein the contact surface (3) is part of an attachment which is detachable from the operating head (1).

14. The device according to any one of the preceding claims, wherein the detection device (5) has a skin detection sensor (16) to determine whether the surface lying on the contact surface (3) is a skin surface or is not a skin surface.

15. The device according to the preceding claim, wherein the skin detection sensor is designed to work contact-free, in particular, as a capacitive and/or optical sensor.

16. The device according to any one of the preceding claims, wherein a movement sensor (17), in particular a speed sensor, is provided to detect the movement, in particular the speed, of the operating head (1) over the surface to be treated.

17. The device according to the preceding claim, wherein the movement sensor (17) is designed to work mechanically, optically and/or capacitively.

18. The device according to any one of the preceding claims, wherein a control unit is provided to control the radiation emitted by the radiation transmitter (2) dependent upon a movement parameter of the operating head (1).

19. The device according to any one of the preceding claims, wherein the device has a fluid applicator for applying a liquid to the skin.

## Revendications

1. Dispositif de réduction de croissance et/ou élimination des poils du corps comprenant une tête de travail (1), qui présente un émetteur de rayonnement (2) pour exposer la surface cutanée à traiter à un rayonnement électromagnétique, ainsi qu'une surface d'appui (3) entourant l'émetteur de rayonnement (2) pour placer la tête de travail (1) sur la surface cutanée, ainsi qu'un dispositif de sécurité (4) pour la commande du rayonnement et/ou de la génération d'un signal de sécurité, qui présente un dispositif de saisie (5) pour la saisie de la pose de la tête de travail (1) sur la surface cutanée, **caractérisé en ce que** la surface d'appui (3) délimite un espace de bruit (6) et le dispositif de saisie (5) présente un dispositif détecteur d'étanchéité (7) pour déterminer l'étanchéité au bruit de l'espace de bruit (6).

2. Dispositif selon la revendication 1, dans lequel le dispositif détecteur d'étanchéité (7) présente, sur un côté de la surface d'appui (3), un générateur de bruit (11) et sur un côté opposé de la surface d'appui (3) un détecteur de bruit (12).

3. Dispositif selon la revendication précédente, dans lequel le générateur de bruit (11) est agencé à l'extérieur de l'espace de bruit (6) et le détecteur de bruit (12) à l'intérieur de l'espace de bruit (6).

4. Dispositif selon une des deux revendications précédentes, dans lequel le générateur de bruit (11) est dirigé vers un bord de la surface d'appui (3).

5. Dispositif selon l'une des revendications 2 à 4, dans lequel plusieurs générateurs de bruit (11) sont prévus sur différentes sections de la surface d'appui (3).

6. Dispositif selon une des revendications précédentes, dans lequel le dispositif détecteur d'étanchéité (7) peut être connecté avec une unité d'évaluation (13) pour la détermination de modifications dans l'étanchéité déterminée de l'espace de bruit (6).

7. Dispositif selon la revendication précédente, comprenant l'unité d'évaluation (13), dans lequel l'unité d'évaluation (13) est prévue pour déterminer une augmentation du niveau de bruit.

8. Dispositif selon la revendication précédente, dans lequel l'unité d'évaluation (13) présente une unité de comparaison (14) conçue pour comparer une modification déterminée du niveau de bruit avec une valeur de seuil prédéfinie et, lors du dépassement de cette valeur de seuil, générer un signal de sécurité.

9. Dispositif selon une des revendications précédentes, dans lequel le dispositif de sécurité (4) peut être connecté avec un dispositif de commande (15) pour la réduction et/ou l'arrêt du rayonnement émis par l'émetteur de rayonnement (2) en fonction d'un signal du dispositif détecteur d'étanchéité (7).

10. Dispositif selon une des revendications précédentes, dans lequel la surface d'appui (3) est conçue pour être souple, en particulier apte à la compression, de préférence à la manière d'un coussin ou d'un soufflet.

11. Dispositif selon une des revendications précédentes, dans lequel la surface d'appui (3) est formée, au moins partiellement, par un anneau saillant avec un côté avant dont le profil peut être adapté.

12. Dispositif selon une des deux revendications précédentes, dans lequel le dispositif détecteur d'étanchéité (7) est placé en-dessous de la surface d'appui (3).

13. Dispositif selon une des revendications précédentes, dans lequel la surface d'appui (3) forme une partie d'un élément détachable de la tête de travail (1).

14. Dispositif selon une des revendications précédentes, dans lequel le dispositif de saisie (5) présente un capteur de détection de la peau (16) pour évaluer la surface supérieure placée sur la surface d'appui (3), pour savoir s'il s'agit d'une surface cutanée ou s'il ne s'agit pas d'une surface cutanée.

15. Dispositif selon la revendication précédente, dans lequel le capteur de détection de la peau est conçu pour fonctionner sans contact, en particulier comme un capteur capacitif et/ou optique.

16. Dispositif selon une des revendications précédentes, dans lequel il est prévu un capteur de mouvement (17), en particulier un capteur de vitesse, conçu pour déterminer le mouvement, en particulier la vitesse, de la tête de travail (1) sur la surface à traiter.

17. Dispositif selon la revendication précédente, dans lequel le capteur de mouvement (17) est conçu pour fonctionner de manière mécanique, optique et/ou capacitive.

18. Dispositif selon une des revendications précédentes, dans lequel un dispositif de commande est prévu pour la commande du rayonnement émis par l'émetteur de rayonnement (2) en fonction d'une valeur de déplacement de la tête de travail (1).

19. Dispositif selon une des revendications précédentes, dans lequel le dispositif présente un applicateur de fluide pour l'application d'un fluide sur la peau.
